# EUROPEAN PATENT APPLICATION

(11) **EP 1 537 890 A1**
(43) Date of publication of application: **08.06.2005**
(21) Application number: 04026997.9
(22) Date of filing: 12.11.2004
(51) Int. Cl.: A61M 25/06, A61M 5/32

(54) **Medical needle device with automatic shielding**

(30) Priority: 01.12.2003 US 725041
(71) Applicant: Becton, Dickinson and Company, Franklin Lakes, New Jersey 07417-1880 (US)
(72) Inventor: Crawford, Jamieson, New York New York 10025 (US)
(74) Representative: von Kreisler, Alek, Dipl.-Chem.

(57) **Abstract**

A shieldable needle device for shielding used needle cannulas. The device generally includes a hub housing and an elongated shield housing. A needle cannula having a distal puncture tip extends from the hub housing, with at least a portion of the needle cannula extending through the passageway of the shield housing. A biasing member acts on the shield housing to bias the shield housing from a first biased position adjacent the hub housing toward a second position covering the distal puncture tip of the needle cannula. An engagement mechanism extends dorsally from the hub housing and is in releasable engagement with a portion of the shield housing for releasably retaining the shield housing in the first biased position adjacent the hub housing. Activation of the engagement mechanism to shield the needle can be effectively accomplished during removal of the needle device from a patient, thereby providing for a passive activation of the device during normal use.

## Description

### BACKGROUND OF THE INVENTION

### 1. Field of the Invention

The present invention relates to a medical device for safe and convenient handling of used needle cannulas. More particularly, the present invention relates to a medical needle device that is adapted to automatically shield a needle cannula during normal use in a medical procedure.

### 2. Description of Related Art

Blood collection sets or intravenous (IV) infusion sets typically include a needle cannula having a proximal end, a distal end with a puncture tip, and a lumen extending therebetween. The proximal end of the needle cannula is mounted to a plastic hub having a central passage that communicates with the lumen in the needle cannula. A thin flexible thermoplastic tube is connected to the hub and communicates with the lumen in the needle cannula. The end of the plastic tube remote from the needle cannula may include a fixture for connecting the needle cannula to a separate medical device, such as a holder, a blood collection tube, and the like.

In order to reduce the risk of incurring an accidental needle-stick wound, protection of used needle cannulas becomes important. With concern about infection and transmission of diseases, methods and devices to cover used needle cannulas have become important and in great demand. For example, some needle assemblies commonly employ a safety shield that may be moved into shielding engagement with a used needle cannula without risking an accidental needle stick.

U.S. Patent No. 5,505,711 discloses a winged injector needle assembly including a needle cannula attached to a hub and a holder forming a shield portion. The hub is slidable within the shield portion. The needle assembly also includes a latching mechanism between the hub and the shield portion to maintain the hub in a forward position with the needle cannula extending from the shield portion. Upon grasping of and release of the latching mechanism, the shield portion may be moved forward to shield the needle cannula. U.S. Patent No. 5,928,199 discloses a similar forward shielding needle assembly, which further includes a lock for locking the shield portion in the forward position shielding the needle cannula. Release of the latching mechanism is awkward due to the relation between the latching mechanism extending between the shield portion and the hub. Further, this device requires the operator to exert a substantial force to shield the needle cannula, and is prone to rotation of the needle cannula within the shield portion during the shielding operation.

U.S. Patent Nos. 5,779,679 and 6,210,371 to Shaw disclose winged IV sets with a retracting needle assembly adapted to automatically retract a needle cannula within a safety shield upon release of a latch. The winged IV sets include a pair of latch wings associated with an outer shield that hold the needle assembly in an unretracted position, and which may be released to cause the needle assembly to be retracted rearwardly within the outer shield. A spring drives the needle assembly rearward to the retracted position within the safety shield. However, since retraction of such a device is achieved by holding the safety shield, the user does not hold the needle cannula during retraction and therefore does not maintain control over the needle cannula. Thus, if the latch is released while the needle cannula is inserted in a patient or during withdrawal from the patient, the spring force may cause an undesirable partial or full retraction of the needle cannula, instead of merely passively activating the device to allow for complete retraction upon full removal from the patient.

In view of the foregoing, there is a continuing need for a shielding medical needle device adapted to shield a used needle cannula once a medical procedure is completed.

### SUMMARY OF THE INVENTION

The present invention relates generally to a safety needle system in the form of a shielding medical needle device for shielding used needle cannulas. The shielding medical needle device generally includes a hub housing and an elongated shield housing. A needle cannula having a distal puncture tip extends from the hub housing, with at least a portion of the needle cannula extending through the passageway of the shield housing. A biasing member such as a compression spring acts on the shield housing to bias the shield housing from a first biased position adjacent the hub housing toward a second position covering the distal puncture tip of the needle cannula. An engagement mechanism extends dorsally from the hub housing and is in releasable engagement with a portion of the shield housing for releasably retaining the shield housing in the first biased position adjacent the hub housing. Activation of the engagement mechanism releases the engagement mechanism from engagement with the shield housing. As such, the shield housing is released from the biased position, with the biasing member causing the shield housing to move toward the second position. Since the engagement mechanism extends dorsally from the device, activation of the device to shield the needle can be effectively accomplished during removal of the needle device from a patient, thereby providing for a passive activation of the device during normal use.

Desirably, the engagement mechanism is comprised of a first member extending dorsally from the hub housing, and a second member extending from a portion of the first member moveable with respect to the first member, such as through a fulcrum. The second member releasably engages a portion of the shield housing, such as through a hook element engaged within a recess or opening which extends through a latch element extending dorsally from the shield housing. Activation of the mechanism can be accomplished by movement of the second member with respect to the first member about the fulcrum, so as to release the hook element from engagement within the opening of the latch element of the shield housing.

Desirably, the needle system includes a pair of flexible wings extending from opposing lateral sides of the hub housing. In such an arrangement, the engagement mechanism extending dorsally from the hub housing desirably bisects the flexible wings extending from opposing lateral sides of the hub housing, but bending of the flexible wings toward a dorsal position does not cause activation of the engagement mechanism.

Additionally, the safety needle system may include a locking mechanism for locking the shield housing in the shielding position, such as by providing the hub housing with at least one flexible cut out portion along a wall thereof for engagement with a portion of the shield housing when the shield housing is in the second position to prevent a return movement of the shield housing to the first position.

In a further embodiment, the present invention is directed to a method for passively activating a safety needle system. The method includes providing a safety needle system comprising a hub housing including a needle cannula extending from a distal end of the hub housing toward a distal puncture tip; a shield housing covering at least a portion of the needle cannula adjacent the hub housing; a biasing element for biasing the shield housing toward a shielding position fully covering the distal puncture tip of the needle cannula; and a latch mechanism extending dorsally from the safety needle system for maintaining the shield housing in a biased state adjacent the hub housing. The method further includes inserting the safety needle system into a patient, and withdrawing the safety needle system from the patient by grasping the latch mechanism, thereby releasing the shield housing from the biased state and causing the shield housing to extend toward the shielding position. Desirably, the hub housing further includes a pair of flexible wings extending from opposing lateral sides thereof. In such an arrangement, the inserting step may include bending the flexible wings to a dorsal position for guiding the needle cannula into the patient. Such bending of the flexible wings to a dorsal position does not cause the latch mechanism to release the shield housing from the biased state. Further, the shield housing may be prevented from re-exposing the distal puncture tip of the needle cannula once the shield housing has been extended to a fully shielding position, such as through a locking mechanism.

In yet a further embodiment, the present invention is directed to a safety needle system which includes a grippable dorsal housing having distal and proximal dorsal housing portions. The proximal dorsal housing portion supports a needle, and the distal dorsal housing portion extends in an axial direction with respect to the needle from a first position adjacent the proximal housing portion to a second position covering a distal tip of the needle. A planar wing structure is integral to at least one of the proximal and/or distal dorsal housing portions, and extends in a direction which is generally normal to the grippable dorsal housing. A biasing element such as a spring extends between the distal and proximal dorsal housing portions for biasing the dorsal housing portion toward the second position. A release element, such as a release latch on the proximal dorsal housing portion in interference engagement with a recess or opening in the distal dorsal housing portion, selectively retains the distal dorsal housing portion in the first position adjacent the first housing portion against the bias of the biasing element. Activation of the release element causes the biasing element to act on the distal dorsal housing portion to move the distal dorsal housing portion to the second position.

Further details and advantages of the present invention will become apparent from the following detailed description when read in conjunction with the drawings, wherein like parts are designated with like reference numerals and lower case letters are included where necessary to identify specific embodiments of the invention.

### DESCRIPTION OF THE DRAWINGS

FIG. 1 is a perspective view of the safety needle assembly of the present invention in the form of a blood collection set, shown in a ready-for-use position with a packaging cover removed and the needle shield retracted to expose the puncture tip of the needle;

FIG. 2 is an exploded perspective view of the assembly of FIG. 1;

FIG. 3 is a perspective view of the needle assembly of FIG. 1 shown with the laterally extending wing members bent into a dorsal position for use during insertion of the needle assembly;

FIG. 4 is a perspective view of the needle assembly of FIG. 1 shown in a fully shielding position covering the puncture tip of the needle;

FIG. 5 is a side view of the needle assembly of FIG. 1 with the needle shield in a retracted position;

FIG. 6 is a side view of the needle assembly of FIG. 4 with the needle shield in a fully shielding position;

FIG. 7 is a top view of the needle assembly of FIG. 1 with the needle shield in a retracted position;

FIG. 8 is a top view of the needle assembly of FIG. 4 with the needle shield in a fully shielding position;

FIG. 9 is a top cross-sectional view of the needle assembly of FIG. 1 with the needle shield in a retracted position;

FIG. 10 is a top cross-sectional view of the needle assembly of FIG. 4 with the needle shield in a fully shielding position;

FIG. 11 is a side cross-sectional view of the needle shield in use with a needle in a retracted position;

FIG. 12 is a side cross-sectional view of the needle shield in use with a needle in a fully shielding position; and

FIG. 13 is a perspective view of a needle clip for use in one embodiment of the present invention.

### DETAILED DESCRIPTION

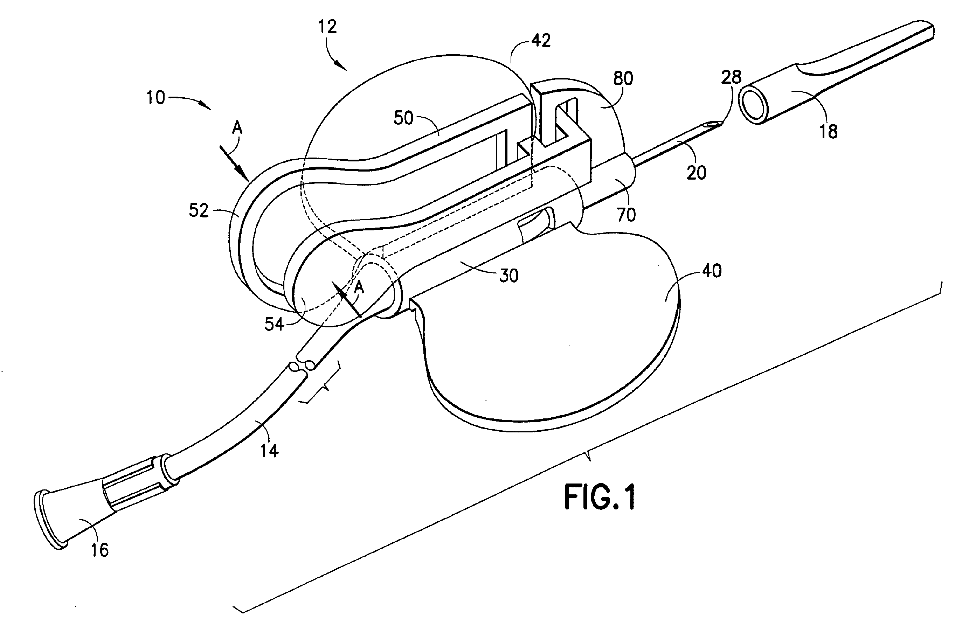

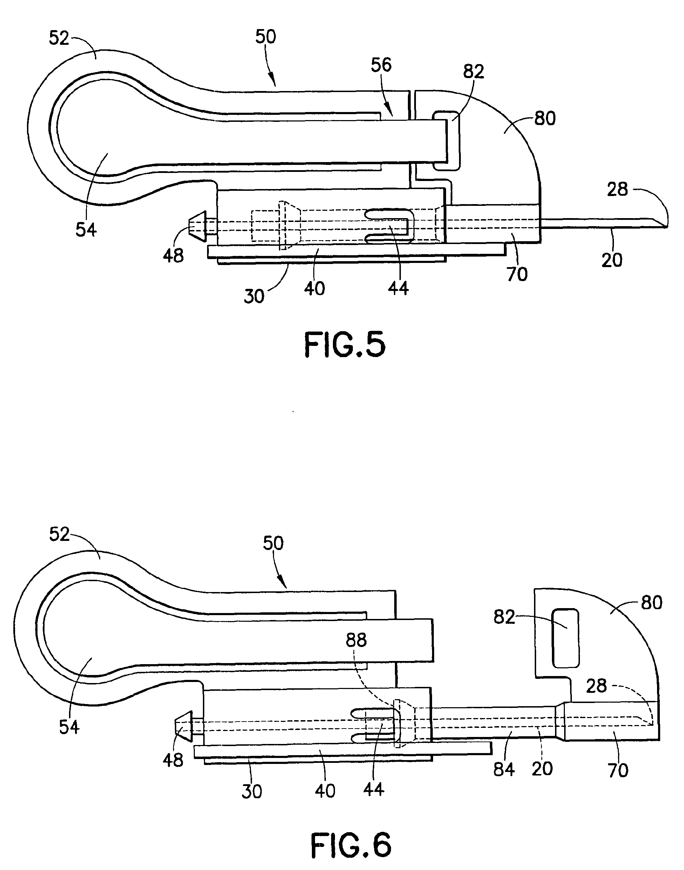

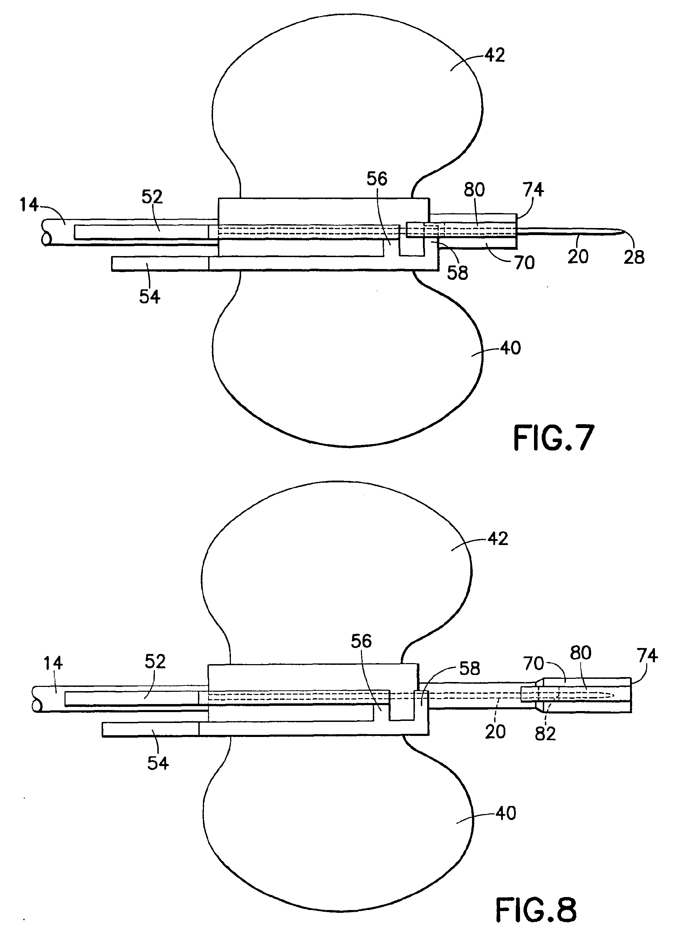

FIGS. 1-10 generally illustrate a medical device according to the present invention. The medical device of the present invention generally includes a shieldable safety needle system adapted to enclose or surround a used needle cannula at the end of a medical procedure, such as a blood collection procedure. The medical device may be used as part of a fluid collection set used to collect blood or other fluids from the body of a human or animal. While described herein in terms of a blood collection set **10**, it is noted that the medical device of the present invention including the safety needle system may be used with any medical device incorporating a needle, such as a syringe system, a double-ended phlebotomy needle, or the like. Preferably, the medical device includes a portion that is automatically biased to a safety, needle-enclosing position during normal use by a user of the medical device resulting in a passive activation of the safety features of the assembly, as discussed in detail hereinafter.

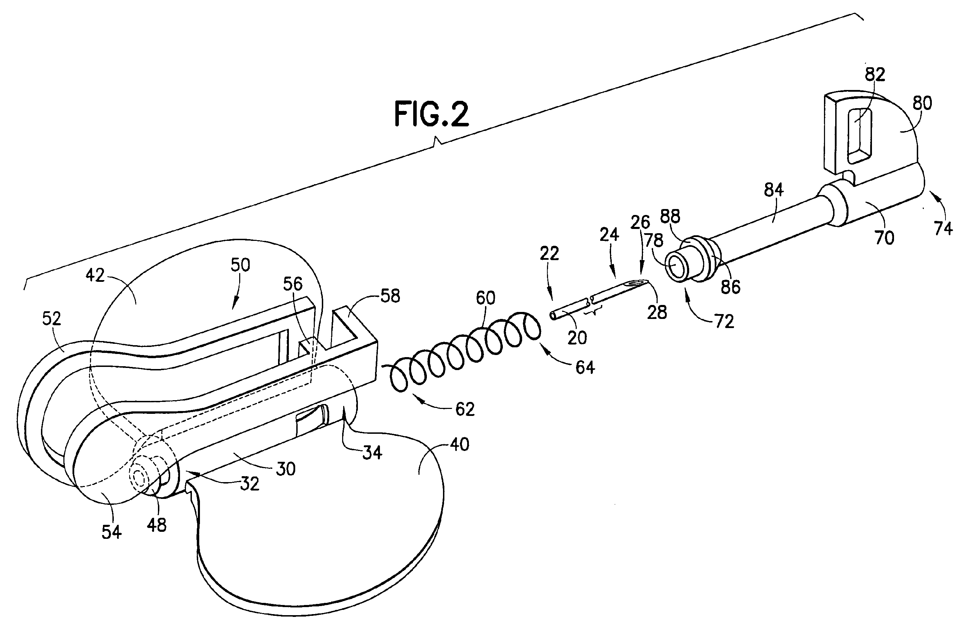

Referring to FIGS. 1 and 2, a first embodiment of the medical device is shown in the form of blood collection set **10**. Blood collection set **10** generally includes a shieldable needle device **12**, a flexible tube **14** extending from needle device **12**, a fixture **16** mounted to tube **14**, and a packaging cover **18** removably mounted to portions of needle device **12** opposite tube **14**, such as through a frictional engagement. Shieldable needle device **12** of blood collection set **10** is shown in detail in FIGS. 2-10, and generally includes a needle cannula **20**, a hub housing **30**, a shield housing **70**, a biasing element in the form of spring **60** biasing the shield housing away from the hub housing, and a latch mechanism **50** for maintaining the shield housing **70** in a biased state adjacent hub housing **30**. The hub housing **30** is adapted for connection to a receptacle (not shown) of, for example, a blood collection set, by way of fixture **16** extending from hub housing **30** through flexible tube **14** by means and procedures known in the art.

Needle cannula **20** includes a proximal end **22** and an opposing distal end **24**, with lumen **26** extending through needle cannula **20** from proximal end **22** to distal end **24**. Distal end **24** of needle cannula **20** is beveled to define a sharp puncture tip **28,** such as an intravenous puncture tip. Puncture tip **28** is provided for insertion into a patient's blood vessel, such as a vein, and is therefore designed to provide ease of insertion and minimal discomfort during venipuncture. Packaging cover **18** is positioned over the distal end **24** of the needle cannula **20** as a removable protective cover to prevent accidental needle-stick wounds prior to using the blood collection set **10** in a medical procedure and to protect the puncture tip **28** prior to use.

Needle assembly **12** further includes hub housing **30**. Hub housing **30** is a unitary structure, desirably molded from a thermoplastic material. Hub housing **30** includes a first, open proximal end **32**, a second, open distal end **34**, and is defined by a rigid tubular wall **36** extending from proximal end **32** to distal end **34**. Tubular wall **36** is characterized by an internal passage **38** extending therethrough from proximal end **32** to distal end **34** of hub housing **30**. Hub housing **30** includes a nub **48** extending from proximal end **32** thereof, which provides structure for connection of flexible tube **14** thereto. Alternatively, hub housing **30** may incorporate structure for attachment to or engagement with an alternate medical device. For example, hub housing **30** may include a luer fitting, such as a female tapering surface at the proximal end thereof for engagement with a male tapering surface of a separate medical device, such as a syringe barrel.

Hub housing **30** further includes a pair of stabilizers in the form of flexible wings **40** and **42** extending laterally from tubular wall **36** at opposing sides thereof, desirably forming a planar wing structure. Wings **40** and **42** provide hub housing **30**, and needle assembly **12**, as a butterfly-type wing assembly, for assistance in positioning and placement of needle assembly **12** during a blood collection procedure, and provide a surface for securing needle device **12** to the skin of a patient, such as through taping wings **40** and **42** to the patient's skin. Wings **40** and **42** may be integrally formed with hub housing **30**, or may be a separate component or components affixed or adhered to hub housing **30**. Wings **40** and **42** may be constructed of a flexible material such that at least one, and preferably both, of the wings **40**, **42** may be bent toward each other and brought together between the fingers of the user to assist in positioning and placing the needle device **12** during venipuncture.

The tubular wall **36** of hub housing **30** includes a pair of cutaway portions adjacent distal end **34** in the form of flexible fingers **44** and **46**. Flexible fingers **44, 46** are cutaway sections of tubular wall **36** and are biased radially inwardly of tubular wall **36** into internal passage **38**. As stated, the tubular wall **36** of hub housing **30** is molded of a thermoplastic material, thereby making the flexible fingers **44** and **46** inherently flexible. Flexible fingers **44, 46** provide needle assembly **12** with a locking mechanism for preventing re-exposure of puncture tip **28** of needle cannula **20** from shield housing **70,** as will be described in more detail herein.

Needle cannula **20** is positioned within internal passage **38** of hub housing **30**, and extends from distal end **34** of hub housing **30**. Desirably, needle cannula **20** and hub housing **30** are separate parts which are fixedly attached and secured through an appropriate medical grade adhesive or the like. More particularly, the proximal end **22** of needle cannula **20** is received within the internal passage **38** of hub housing **30**, with the distal end **24** of needle cannula **20** projecting outward from the distal end **34** of the hub housing **30**. The internal passage **38** communicates with the lumen **26** defined in the needle cannula **20** to enable fluid, such as blood, to pass through the needle device **12** and to the flexible tube **14** connecting the needle device **12** to the blood collection receptacle. The needle cannula **20** is preferably secured adjacent the proximal end **32** of hub housing **30** through the use of an appropriate medical grade adhesive, mechanical means, or the like.

A latch mechanism **50** extends dorsally from the top surface of hub housing **30**. Latch mechanism **50** includes a first member and a second member **54** extending from a portion of the first member **52**. First member **52** and second member **54** form generally elongated finger tabs which extending axially toward the proximal position, i.e., toward proximal end **32** of hub housing **30**. Second member **54** includes a forward or distally extending portion with a hook **58** at a distal end thereof, and extends from first member **52** through a lever arm which creates a fulcrum **56.** As such, first member **52** and second member **54** can be squeezed or pinched toward each other about fulcrum **56**, which causes hook **58** at the distal portion of second member **54** to pivot outwardly.

As noted, wings **40** and **42** extend from opposing lateral sides of hub housing **30**, generally in a planar fashion. Latch mechanism **50** extends dorsally from the top surface of housing **30** and generally bisects the plane defining wings **40** and **42**, such that wings **40**, **42** are generally normal to latch mechanism **50**. Moreover, first member **52** and second member **54** of latch mechanism **50** extending in a generally proximal direction such that bending together of wings **40** and **42** in a dorsal manner does not cause first member **52** and second member **54** to be squeezed or pinched together due to the positioning of the finger tabs of first member **52** and second member **54** and of the lever arm or fulcrum **56**, as will be discussed further with respect to use of the device.

Needle device **12** further includes shield housing **70**. The shield housing **70** extends between a first, open proximal end **72** and a second, open distal end **74**, and is defined by a generally hollow tubular wall **76** having an internal passage **78.** Shield housing **70** is preferably a unitary structure, which is desirably molded from a thermoplastic material. The internal passage **78** is sized to coaxially receive needle cannula **20**, with shield housing **70** axially slidable thereabout. As such, shield housing **70** is movable along needle cannula **20** between a first proximal or retracted position adjacent hub housing **30**, and a second distal position encompassing and shielding puncture tip **28,** as will be described in more detail herein.

The proximal end **72** of shield housing **70** is sized to be received within the distal end **34** of hub housing **30** and extend within internal passage **38** thereof. Accordingly, tubular wall **76** of shield housing **70** may include a portion of reduced diameter at stepped down portion **84**, which extends toward the proximal end **72** of shield housing **70**. This stepped down portion **84** slidably cooperates within the internal passage **38** of hub housing **30**. Shield housing **70** may further include a neck **86** extending radially about the stepped down portion **84,** forming a shoulder **88** facing the proximal end **72** of shield housing **70** for interference engagement with flexible fingers **44, 46** to providing a locking mechanism, as will be discussed in more detail herein.

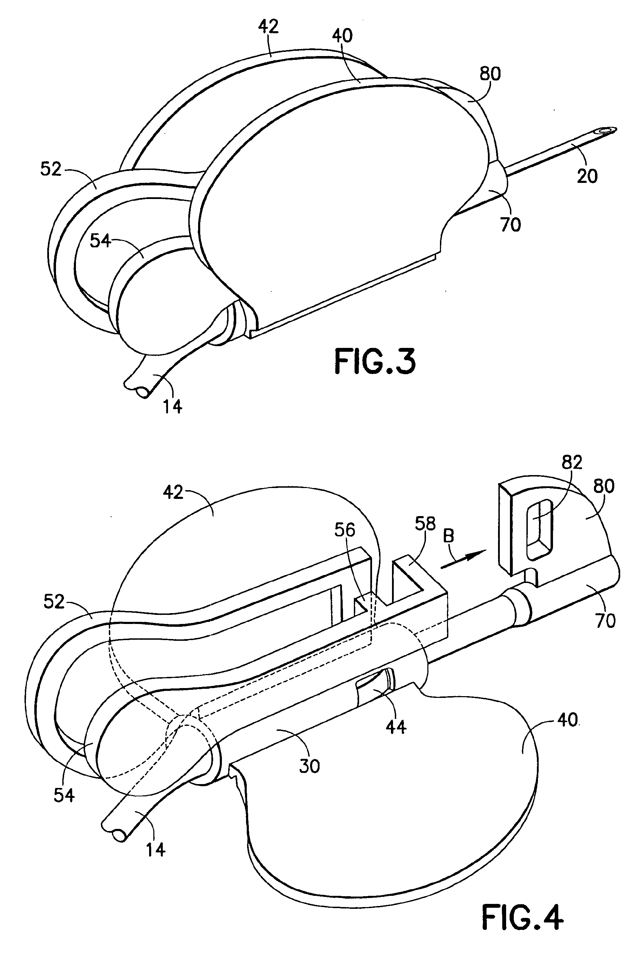

Shield housing **70** is generally movable between a first or retracted position adjacent hub housing **30** with at least a portion of shield housing **70**, such as proximal end **32** thereof, coaxially contained within internal passage **38** of hub housing **30**, and a second, extended or needle-shielding position, wherein the needle cannula **20** is enclosed or disposed within shield housing **70** fully covering the needle cannula **20** and, in particular, the puncture tip **28**. The first or retracted position of the shield housing **70** is shown in FIGS. 1, 5, 7 and 9 and the second or extended position of the shield housing **70** is shown in FIGS. 4, 6, 8 and 10. In the first retracted position, the distal end **24** of needle cannula **20** projects outward from the shield housing **70** for use in a fluid collection procedure.

A latch element **80** extends dorsally from the top surface of shield housing **70**, forming a dorsal wing-like structure. Latch element **80** includes a recessed portion within the surface thereof, or an opening such as opening **82** extending through the surface thereof. Opening **82** of latch element **80** is provided for establishing releasable engagement with hook **58** of latch mechanism **50** of hub housing **30**. As such, the engagement between hook **58** of latch mechanism **50** within recess or opening **82** of latch element **80** establishes a dorsally extending engagement mechanism for releasably retaining shield housing **70** in the proximal or retracted position adjacent hub housing **30**.

The needle device **12** preferably further includes a biasing member such as compression spring **60** extending between the hub housing **30** and the shield housing **70**. Spring **60** is adapted to act on the shield housing **70** to bias the shield housing **70** from the first retracted position adjacent hub housing **30** toward the second shielding position in which shield housing **70** encompasses puncture tip **28** of needle cannula **20**. The biasing member or spring **60** is disposed within internal passage **38** of hub housing **30** about needle cannula **20**, and extends between the internal surface within internal passage **38** at the proximal end **32** of hub housing **30** and shoulder **88** of neck **86** extending circumferentially about the proximal end of shield housing **70** extending within hub housing **30**. Spring **60** is maintained in a compressed state by engagement of the hook **58** of the second member **54** of latch mechanism **50** within the recess or opening **82** in the latch element **80** of shield housing **70**. If desired, the proximal end **62** of the spring **60** may be secured to the proximal end **32** of hub housing **30** by a suitable medical grade adhesive known in the art or by a fixed mechanical connection. Likewise, the distal end **64** of the spring **60** may be adhesively or mechanically secured to the shield housing **70**, such as at shoulder **88**. Once the spring **60** is released by disengaging the hook **58** from the corresponding opening **82**, the spring **60** exerts an axial expansion force against the shield housing **70**, and in particular, against the proximal end **72** or shoulder **88** of shield housing **70**, relative to the hub housing **30**. The shield housing **70** is urged by the spring **60** to the second, needle-shielding position, wherein the shield housing **70** covers the needle cannula **20** (i.e., FIG. 4). The spring **60** also substantially prevents the shield housing **70** from moving proximally back toward the first position within hub housing **30** by providing a counter-acting biasing force against such a movement.

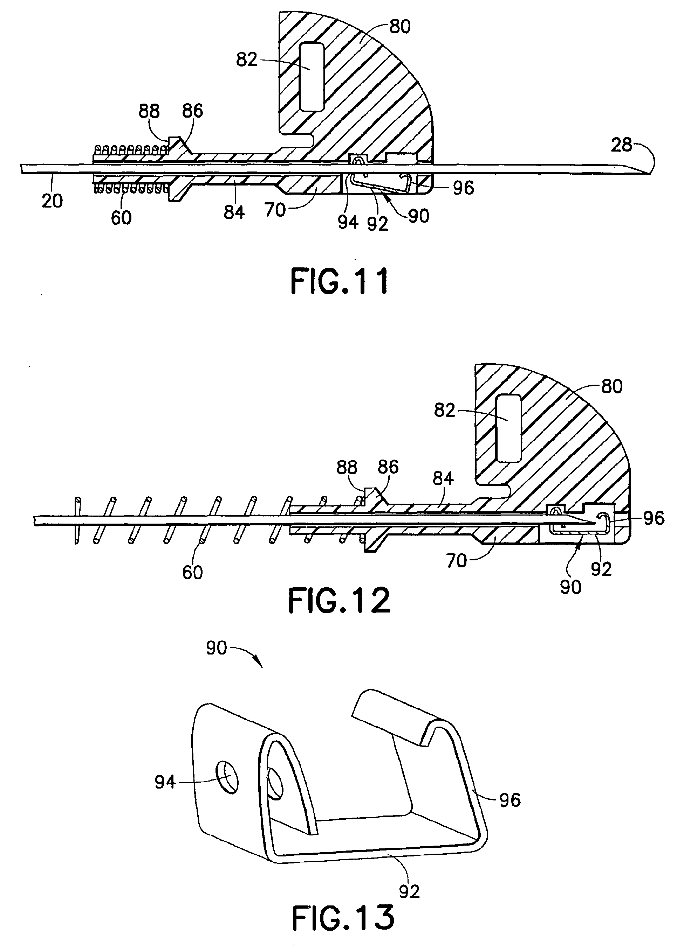

In one embodiment, shield housing **70** may further include a protective clip **90** for locking out puncture tip **28** of needle cannula **20** and preventing re-exposure thereof. As best seen in FIGS. 11-13, clip **90** may be unitarily stamped and formed from a resiliently deflectable metallic material. Clip **90** includes a planar spring leg **92** extending between a proximal end and an opposed distal end. A mounting aperture **94** extends through clip **90** at a location near the proximal end. A lock out leg **96** extends angularly from the distal end of clip **90,** and is bent back toward the proximal end of clip **90**. The bends in lock out leg **96** enable secure protective engagement with puncture tip **28** of needle cannula **20** and further enable smooth axial sliding movement of shield housing **70** along needle cannula **20**, as described in further detail herein.

In use, blood collection set **10** is provided with needle device **12** assembled and including flexible tube **14** extending from needle device **12** and connected to fixture **16.** Blood collection set **10** is preferably packaged with the shield housing **70** in the first retracted position, as shown, for example, in FIG. 1. After removing blood collection set **10** from its package, it can be assembled with other appropriate medical equipment for use. For example, a non-patient needle assembly and a needle holder may be connected to blood collection set **10** through fixture **16**.

To prepare for use of blood collection set **10**, the user grasps wings **40** and **42** at opposing lateral sides of hub housing **30** between a thumb and forefinger, and bends wings **40** and **42** dorsally toward each other, with latch mechanism **50** extending therebetween, as shown in FIG. 3. Packaging cover **18** is then grasped and urged distally to disengage from needle cannula **20** and/or shield housing **70**, thereby exposing puncture tip **28** of needle cannula **20**.

The overall design and profile of latch mechanism **50** is configured such that bending of the wings **40** and **42** to the dorsal position for positioning and placement does not cause activation of the latch mechanism **50**. In other words, bending of wings **40** and **42** together does not cause pinching movement of the finger tabs of first member **52** and second member **54** relative to each other, which would disengage the hook **58** from interference engagement with opening **82**. This may be accomplished by maintaining the lever arm or fulcrum **56** at a position directly adjacent the point of contact between bent wings **40** and **42.** As such, shield housing **70** is maintained in the first retracted position with latch mechanism **50** unactivated during bending of wings **40** and **42** for positioning and placement of needle device **12.** Desirably, wings **40** and **42** are of sufficient length to extend dorsally beyond latch mechanism **50** when wings **40** and **42** are bent together, thereby providing further assurance that latch mechanism **50** will not be activated to release shield housing **70** during positioning and placement.

After packaging cover **18** is removed, the medical practitioner can then urge puncture tip **28** at distal end **24** of needle cannula **20** into a targeted blood vessel of a patient with wings **40** and **42** bent inwardly toward each other between the user's fingers to act as a structure for guided placement, as is known in the art. After the targeted blood vessel has been accessed, the medical practitioner can release the grip on wings **40** and **42**, and wings **40** and **42** can thereafter be taped to the patient's skin to prevent movement of the needle device **12** during use.

Upon completion of the procedure, such as when all desired samples have been drawn, needle cannula **20** can be withdrawn from the patient. As opposed to conventional techniques for withdrawing wingsets by again bending the wings together in a dorsal manner, withdrawal of needle device **12** is preferably accomplished by grasping latch mechanism **50**, that is by grabbing the finger tabs of first member **52** and second member **54** between a user's thumb and forefinger. This grabbing causes first member **52** and second member **54** to move toward each other in a direction of arrows A in FIG. 1, with fulcrum **56** acting as a pivot point for movement thereof. This movement toward each other in turn causes the distal end portion of second member **54** to pivot outwardly, which causes hook **58** to disengage from interference engagement within opening **82** of latch element **80** of shield housing **70**. Once the interference engagement between hook **58** and opening **82** is released, the bias from spring **60** causes shield housing **70** to be propelled distally in a direction of arrow **B** in FIG. 4, away from hub housing **30** along needle cannula **20**. At this point, the puncture tip **28** of needle cannula **20** may still be positioned within the patient. As such, shield housing **70** is propelled forward to a position in which it contacts the patient's skin. The user can then remove the needle device **12** from the patient, during which the shield housing **70** continues to move forward under the bias of the spring **60**, until the needle cannula **20** is fully withdrawn from the patient, at which point the shield housing **70** extends to the fully shielding position completely encompassing puncture tip **28**. After shield housing **70** is moved along needle cannula **20** past the puncture tip **28**, lockout leg **96** of clip **90** will pass distally beyond puncture tip **28** of needle cannula **20**. The inherent resiliency of spring leg **92** of clip **90** will urge lockout leg **96** over puncture tip **28** of needle cannula **20**. Thus, a return movement of shield housing **70** is prevented through clip **90** acting as a locking mechanism. Hence, puncture tip **28** of needle cannula **20** is safely shielded. Blood collection set **10** may then be appropriately discarded.

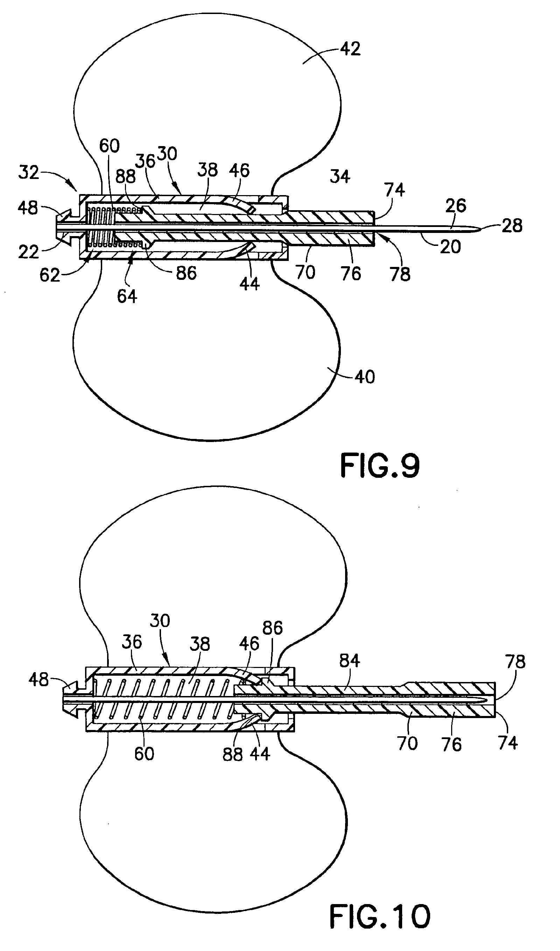

In addition to or instead of the locking mechanism established through clip **90**, a further locking mechanism may be provided through the interference interaction between flexible fingers **44, 46** and shoulder **88** of neck **86** on shield housing **70**. More particularly, as spring **60** propels shield housing **70** distally in the direction of arrow **B**, neck **86** of shield housing **70** passes beyond flexible fingers **44, 46**, which are biased radially inwardly against the outer surface of shield housing **70**. Once neck **86** passes beyond flexible fingers **44, 46,** the flexible fingers **44, 46** abut against shoulder **88**, thereby preventing shield housing **70** from movement backward in the opposite direction, as seen in FIG. 10. As such, shield housing **70** is locked in the shielding position encompassing puncture tip **28**. It will be appreciated that the location of the flexible fingers and corresponding shoulder may be reversed in accordance with the present invention. In addition, neck **86** of shield housing **70** may be trapped by the distal end of hub housing **30** to prevent movement beyond hub housing **30,** such as through a return portion within the distal end **34** of hub housing **30** which prevents the passage of neck **86** therebeyond.

Alternatively, if desired, activation of latch mechanism **50** to release shield housing **70** can be accomplished immediately after needle cannula **20** is placed within the patient's blood vessel, as opposed to waiting for withdrawal of needle device **12** from the patient.

Alternatively, activation of latch mechanism **50** may be delayed until after needle cannula **20** is fully removed from the patient. For example, wings **40** and **42** may be bent together in a dorsal manner to create a structure for holding needle device **12** and withdrawing needle cannula **20** from the patient. After the needle cannula **20** is withdrawn in this manner, activation of latch mechanism **50** can be accomplished to release shield housing **70** and effectively shield the used puncture tip **28**. As such, the needle device of the present invention is selectively passive.

The inner surface of the hub housing **30** and the outer surface of the shield housing **70** may interact such that any axial rotation of the hub housing **30** with respect to the shield housing **70** is inhibited during the sliding movement of the shield housing **70** from the first retracted position to the second shielding position. For example, a surface feature may be provided on one of the outer surface of the shield housing **70** or the inner surface of the hub housing **30**, such as a plurality of spline grooves or detents. Alternatively, the hub housing **30** and shield housing **70** may each have a non-circular cross section, for example, with one planar side, desirably a bottom flat surface, to prevent relative rotation between these elements.

The needle device of the present invention is particularly innovative since it involves a passive activation of the safety shielding feature through the ergonomic layout of the device. In particular, when in the retracted position, the shield housing interengages with the hub housing to form a grippable dorsal housing structure with the hub housing forming a proximal housing structure and the shield housing forming a distal housing structure, and incorporating a latch mechanism extending dorsally therebetween. Moreover, the shield housing is automatically activated to shield the needle cannula during the typical steps involved in a venipuncture procedure. For example, the device is easily insertable within a patient using a normal technique with bent wings forming a dorsal structure for guiding, positioning, and placement of the device. The shield housing can remain retracted during the blood sampling procedure, thereby preventing the shield housing from interfering with the user during the sampling procedure, and preventing the patient from being startled by activation of the shield against the patient's skin prior to the sampling procedure. Moreover, activation is easily accomplished during a typical procedure for removing the needle device from the patient, i.e., by grasping the device in a dorsal manner, through the latch mechanism extending dorsally from the device. This grasping automatically passively activates the device during removal, thereby avoiding any active step for shielding of the assembly by the user, and preventing any unnecessary exposure to the used needle cannula. The selectively passive activation affords a greater degree of safety to the user with minimal or no change in technique, as compared with conventional safety needle mechanisms.

While the medical device of the present invention is described in terms of several preferred embodiments for use in connection with bodily fluid collection systems and like devices, the present invention may take many different forms. The preferred embodiments shown in the drawings and described hereinabove in detail are to be considered as exemplary of the principles of the invention and are not intended to limit the invention to the embodiments illustrated. Various other embodiments will be apparent to and readily made by those skilled in the art without departing from the scope and spirit of the invention. The scope of the present invention will be measured by the appended claims and their equivalents.

## Claims

1. A safety needle system, comprising:
a hub housing having a first end and an opposed second open end with a passageway extending therebetween;
an elongated shield housing having a first open end and an opposed second open end with a passageway extending therebetween;
a needle cannula having a distal puncture tip, the needle cannula extending from the hub housing with at least a portion of the needle cannula extending through the passageway of the shield housing;
a biasing member acting on the shield housing to bias the shield housing from a first biased position adjacent the hub housing toward a second position covering the distal puncture tip of the needle cannula; and
an engagement mechanism extending dorsally from the hub housing, the engagement mechanism in releasable engagement with a portion of the shield housing for releasably retaining the shield housing in the first biased position;
wherein engagement of the engagement mechanism with the shield housing maintains the shield housing in the first biased position adjacent the hub housing, and wherein activation of the engagement mechanism releases the engagement mechanism from engagement with a portion of the shield housing, thereby releasing the shield housing from the biased position and permitting the biasing member to cause the shield housing to move toward the second position.

2. The safety needle system as in claim 1, wherein the engagement mechanism comprises a first member extending dorsally from the hub housing, and a second member extending from a portion of the first member and in engagement with a portion of the shield housing, the second member moveable with respect to the first member so as to release from engagement with the shield housing.

3. The safety needle system as in claim 2, wherein the shield housing includes a latch element extending dorsally from the shield housing, the latch element including a recess or opening therein for engagement with the engagement mechanism of the hub housing.

4. The safety needle system as in claim 3, wherein the second member of the engagement mechanism includes a hook element for engagement with the recess or opening of the latch element.

5. The safety needle system as in claim 4, wherein the second member of the engagement mechanism is connected to the first member of the release arrangement through a fulcrum.

6. The safety needle system as in claim 5, wherein the first and second members of the engagement mechanism include corresponding surfaces for movement toward each other about said fulcrum, thereby causing movement of said hook element out of engagement from said recess or opening of said latch element.

7. The safety needle system as in claim 1, wherein said hub housing further comprises structure for attachment to a medical device.

8. The safety needle system as in claim 1, wherein the biasing member comprises a compression spring.

9. The safety needle system as in claim 1, wherein a portion of the shield housing extends within the passageway of the hub housing.

10. The safety needle system as in claim 9, wherein the hub housing further comprises at least one flexible cut out portion along a wall thereof, said flexible cut out portion biased inwardly toward said passageway of said hub housing.

11. The safety needle system as in claim 10, wherein said flexible cut out portion is adapted for engagement with a portion of said shield housing when said shield housing is in said second position to prevent a return movement of said shield housing to said first position.

12. The safety needle system as in claim 1, further comprising a pair of flexible wings extending from opposing lateral sides of said hub housing.

13. The safety needle assembly as in claim 12, wherein the engagement mechanism extending dorsally from the hub housing bisects the flexible wings extending from opposing lateral sides of the hub housing, and wherein bending of the flexible wings toward a dorsal position does not cause activation of the engagement mechanism.
